# EUROPEAN PATENT APPLICATION

(11) **EP 1 127 486 A1**
(43) Date of publication of application: **29.08.2001**
(21) Application number: 01103596.1
(22) Date of filing: 21.02.2001
(51) Int. Cl.: A01K 67/027

(54) **A method for production of a transgenic animal**

(30) Priority: 24.02.2000 JP 2000047627; 21.11.2000 JP 2000354339
(71) Applicant: Kyoto University, Kyoto (JP)
(72) Inventor: Nakatsuji, Norio, Kyoto City, Kyoto (JP); Tamura, Masaru, Mishima City, Shizuoka Pref. (JP); Huang, Zenyong, Shinjuku-ku, Tokyo (JP); Sakurai, Takayuhi, Isehara City, Kanagawa Pref. (JP); Chuma, Shinichiro, 2-D Domiru-ichibankan, Kamigyo-ku, Kyoto City, Kyoto (JP); Saito, Tetsuichiro, 508 Sunlife Takara, Kyoto City, Kyoto (JP)
(74) Representative: Wibbelmann, Jobst, Dr., Dipl.-Chem.

(57) **Abstract**

A novel and efficient method for production of a transgenic animal is developed. This invention provides a method for production of a novel transgenic animal comprising the steps of introducing a foreign gene into testis of an animal, selecting transfected spermatocytes, spermatids or spermatozoa in which die foreign gene was introduced using a fluorescence marker gene as an indicator and fertilizing an unfertilized oocyte or ovum with the transfected spermatocyte, spermatid or spermatozoa.

## Description

### BACKGROUND OF THE INVENTION

### 1. Field of the invention

This invention relates to a novel method for production of a transgenic animal.

### 2. Description of the related art

In the past, micro-injection of DNA into pronuclei of a fertilized ovum has been most conventionally used as a method for introduction of a foreign gene into a mammalian animal. The method is an assured method used for many animal species including domestic animals.

However, the probability of gene introduction according to this method is low. Therefore, using technique of micro-injection into a fertilized ovum, a transgenic animal transfected by a foreign gene could be obtained at efficacy of about 1%. That is, the efficacy of gene transfection achieved by the conventional method was low. Therefore, considering application of micro-injection method to a large animal, the cost necessitate would be very expensive. Moreover, the integration site of a foreign gene on a chromosome can not be regulated and the site of integration is almost random.

On the other side, concerning a method of gene introduction, which comprising treatment of sperm using DNA followed by fertilization, stability and reliability of the method is low. However, the result recently published by the group of Professor Yanagimachi of Hawaii University have attracted attention of researchers. That is, a transgenic mouse was obtained at higher efficacy by mixing frozen sperm of a mouse with DNA followed by microscopic fertilization using intra-cytoplasmic sperm injection. Although it has been examined whether the method is applicable to other mouse breed and other animal species, the range of application of this method is still unknown. Moreover, the integration site of foreign DNA on a chromosome can not be regulated, as the same as the intra-pronuclei injection.

The method of targeted genetic recombination (gene targeting), which can achieve gene destruction by substituting target gene on a chromosome with a foreign vector, can be performed by introduction of a vector containing a portion homologous to endogenous gene, subsequently selection of those incited homologous genetic recombination. Currently, a transgenic mouse individual, containing alteration on certain targeted gene, have been produced by performing gene introduction and selection in embryonic stem-cell (ES cell) lines, followed by production of a chimeric mouse contributing to germ cell line and then mating it.

The efficiency of the method is low and consumes a lot of time and cost. Furthermore, the range of ES cell lines, capable of utilizing for gene targeting, is restricted to only several mouse strains. For domestic animals unable to obtain ES cell lines, another method has been attempted. That is, a method comprising homologous genetic recombination in a somatic-cell line, subsequently performance of animal cloning by nuclear transplantation to a de-nucleated ovum. However, the probability of production of a transgenic animal is unknown.

### SUMMARY OF THE INVENTION

The object of this invention is to provide a convenient method for production of a transgenic animal with high efficacy. Moreover, considering application to production of a transformed domestic animal and the like, the method of this invention should applicable to a large animal.

The method of this invention comprises gene introduction to spermatogenetic cells in testis and it is characteristic for direct gene introduction to a germ cell. Therefore, production of a transgenic animal at high efficiency can be realized by the method of this invention, compared with the above-mentioned conventional method. The inventors remarked a convenient and rapid method of injecting DNA to seminiferous tubules in testis, followed by applying electric pulses to it. Then a method to produce a transgenic mouse was developed. The method of this invention further comprises the steps of screening of transfected spermatocytes, spermatids and spermatozoa with a foreign gene introduced and fertilization of oocyte in vitro.

Incidentally, Yamazaki et al. injected LacZ marker gene, which is detectable by histochemical staining, into mouse testis and electric pulses were applied to it. It was reported that small number of spermatogenic cells, expressing the marker gene, were recognized in the testis two months after application of electric pulses (Yamazaki et al., Biology of reproduction, 59, 1439-1444 (1998)). However, integration of a foreign gene was not confirmed and production of a transgenic mouse individual was not achieved.

This invention is a method for production of a transgenic animal with a gene encoding a fluorescent protein introduced, the method comprising the steps of:
preparing a plasmid into which said gene encoding a fluorescent protein is incorporated in the plasmid ;
introducing said plasmid into germ cells existing in a testis of an animal by electroporation method or lipofection method to prepare transfected spermatocytes, spermatids or spermatozoa in which said gene encoding a fluorescent protein is introduced;
separating said transfected spermatocytes, spermatids or spermatozoa from non-transfected spermatocytes, spermatids or spermatozoa using fluorescence of said fluorescent protein as a marker ;
fertilizing an unfertilized oocyte or ovum with said transfected spermatocyte, spermatid or spermatozoon to prepare a fertilized zygote ; and obtaining a transgenic animal individual from said fertilized zygote.

Moreover, this invention is a method for production of a transgenic animal with an intended foreign gene introduced, the method comprising the steps of:
preparing a plasmid into which said intended foreign gene and a gene encoding a fluorescent protein are incorporated in the plasmid ;
introducing said plasmid into germ cells existing in a testis of an animal by electroporation method or lipofection method to prepare transfected spermatocytes, spermatids or spermatozoa in which said intended foreign gene and said gene encoding a fluorescent protein are introduced;
separating said transfected spermatocytes, spermatids or spermatozoa from non-transfected spermatocytes, spermatids or spermatozoa using fluorescence of said fluorescent protein as a marker ;
fertilizing an unfertilized oocyte or ovum with said transfected spermatocyte, spermatid or spermatozoon to prepare a fertilized zygote ; and obtaining a transgenic animal individual from said fertilized zygote.

These and other features and advantages of this invention will become apparent upon a reading of the detailed description and drawings.

### BRIEF DESCRIPTION OF THE DRAWINGS

Fig. 1 is a scheme figure showing the method for production of a transgenic animal according to this invention,
Fig. 2 is the photograph showing bright field image of spermatocytes isolated from testis, observed 15-18 days after transfection of GFP gene,
Fig. 3 is the photograph showing fluorescence image of spermatocytes isolated from testis, observed 15-18 days after transfection of GFP gene,
Fig. 4 is the photograph showing overlapped image, in which bright field image and fluorescence image were combined, of spermatocytes isolated from testis, observed 15-18 days after transfection of GFP gene,
Fig. 5 is the photograph showing bright field image of spermatids isolated from testis, observed 15-18 days after transfection of GFP gene,
Fig. 6 is the photograph showing fluorescence image of spermatids isolated from testis, observed 15-18 days after transfection of GFP gene,
Fig. 7 is the photograph showing overlapped image, in which bright field image and fluorescence image were combined, of spermatids isolated from testis, observed 15-18 days after transfection of GFP gene,
Fig. 8 is the photograph showing fluorescence image of testis, observed 2 days after transfection of YFP gene,
Fig. 9 is the photograph showing bright field image of testis, observed 2 days after transfection of YFP gene,
Fig. 10 is the photograph showing fluorescence image of seminiferous tubule, observed 2 days after transfection of GFP gene,
Fig. 11 is the photograph showing fluorescence image of seminiferous tubule, observed 18-20 days after transfection of YFP gene,
Fig. 12 is the photograph showing overlapped image, in which bright field image and fluorescence image were combined, of spermatozoa isolated from seminiferous tubule, observed 18-20 days after transfection of YFP gene,
Fig. 13 is the photograph showing bright field image of spermatozoa isolated from seminiferous tubule, observed 18-20 days after transfection of YFP gene,
Fig. 14 is the photograph showing fluorescence image of 8-cells stage embryo fertilized with a sperm transfected by YFP gene,
Fig. 15 is the photograph showing bright field image of 8-cells stage embryo fertilized with a sperm transfected by YFP gene,
Fig. 16 is the photograph showing fluorescence image of fetuses at the 12.5th day of gestation,
Fig. 17 is the photograph showing overlapped image, in which bright field image and fluorescence image were combined, of fetuses at the 12.5th day of gestation,
Fig. 18 is the photograph showing fluorescence image of tails from the individuals obtained,
Fig. 19 is the photograph showing bright field image of tails from the individuals obtained, and
Fig. 20 is the photograph showing detection of YFP gene by Southern blotting analysis.

### DETAILED DESCRIPTION OF THE INVENTION

The inventor attempted introduction of a foreign gene at stages before meiosis or during meiosis. Technique of electroporation into spermatogonia or spermatocytes existing in mouse testis was utilized for it. For the purpose to utilize for in vitro fertilization by intra-cytoplasmic sperm injection, the inventor detected and screened transfected spermatocytes, spermatids or spermatozoa, with a foreign gene introduced. Here, gene encoding a fluorescent protein, such as gene of Green Fluorescent Protein (GFP) or gene of Yellow Fluorescent Protein (YFP), was used as a tool.

The inventor injected DNA into seminiferous tubules of mouse testis at the age of 14 days and optimized parameters of electroporation. Fluorescence of GFP/YFP, exhibiting wide spread expression in germ cells, was stable for 1 week in maximum. The number of cells expressing fluorescence decreased after 1 week of DNA injection.

Cluster of spermatozoa, expressing fluorescence in seminiferous tubule, was obtained 18-20 days after gene introduction. As the result of using such sperm for intra-cytoplasmic sperm injection, fetuses and the pups expressing widespread fluorescence were obtained. In individuals expressing such fluorescence, it was found that the foreign gene was integrated into chromosome, according to the result of Southern-blotting analysis. In this method, 7/8 or more of obtained individuals were transformants. Moreover, it was observed that the transformants were expressing the marker gene in the whole body. The result suggested that probability of transformation was very high and gene introduction already occurred in the spermatogenic cells.

According to the method of this invention, young mouse containing spermatogenic cells up to pachytene stage was used. It was confirmed that there are only spermatogonia and primary spermatocytes at this stage. That is, the method of this invention is a novel method characterized by introducing a foreign vector into germ cell at early stages of meiosis. Since it is the stage that frequent homologous recombination occurs between homologous chromosomes, occurrence of homologous recombination of target gene can be expected at high probability. In the literature of Yamasaki et al. mentioned above, mice of 4 to 6 weeks old are utilized. Compared with it, the method of this invention is characteristic for utilizing a juvenile mouse and increase in the efficiency of gene introduction can be expected.

Such method for transformation according to this invention can be used to introduce other genes by linking to a gene encoding a fluorescent marker. The electroporation method according to this invention is very easy and it can be used for various target animals including domestic animals of large size. This invention provides a method for direct introduction of a foreign gene into germ cells. This alternative method for transformation of a mammalian might provide an useful method bearing various new possibilities.

The method of this invention may be an efficient method for transformation and gene targeting, applicable to a laboratory animal and a domestic animal. The latter possibility is important for production of a pathologic model animal of human disease by destruction of a gene related to onset of the disease. Moreover it is important for development of a donor animal utilized for organ or tissue transplantation, produced by substitution of major-histocompatibility genes.

The features of this invention are as follows.
(1) This invention enables screening of sperm cells transfected by a foreign gene by observation of fluorescence using a marker gene. That is, genetic expression of the marker gene can be detected in the status of alive.
(2) This invention is the first example succeeded in production of a transgenic animal individual by performing in vitro fertilization utilizing a sperm transfected by a foreign gene, which can be performed with high probability. The method of this invention is different from known methods, for this method comprises in vivo integration utilizing a male animal with a foreign gene introduced to its testis. Since germ cells, to which a foreign gene was introduced, can be selected using fluorescence of the marker protein at the status of alive, the transgenic animal can be produced with high probability.
(3) To perform gene introduction according to this invention, testis of a juvenile mouse was chosen, as such testis is assumed to be at the early stage of spermatogenesis.
(4) It is assumed that marker proteins (GFP and YFP) might exhibit some cytotoxic effect in the process of spermatogenesis. Therefore, in many cases, spermatogenic cells expressing fluorescence tend to decrease and disappear drastically, 1 to 3 weeks after transfection. In order to reduce such cytotoxic effect, YFP gene linked to a mitochondrial localization signal peptide was chosen to be introduced.

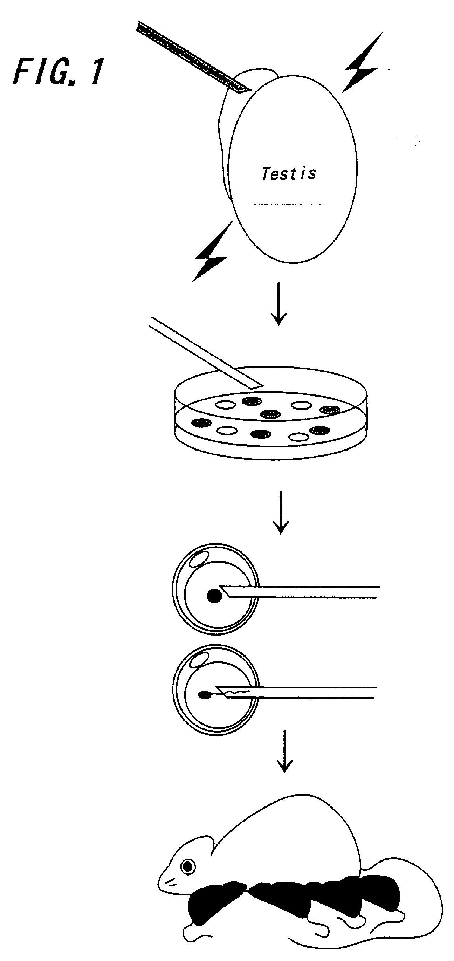

This invention provides a method for production of a transgenic animal comprising following processes. Schematic view of this invention is indicated in Fig. 1.
(1) A plasmid, with a foreign intended gene and a gene encoding a fluorescent protein were integrated, was introduced into germ cells existing in testis of an animal.
(2) Screening of spermatocytes, spermatids or spermatozoa to which the intended gene was introduced, using a fluorescent protein as an indicator.
(3) Fertilization of an unfertilized oocyte or ovum with transfected spermatocyte, spermatid or spermatozoon transfected by intended gene introduced to prepare a fertilized zygote.
(4) Incubation of said fertilized zygotes.

Here, the foreign intended gene and the fluorescent protein gene can be connected into one plasmid or can be transfected as two distinct plasmids separately. According to previous reports, it is known that plural exogenous genes, including genes encoding fluorescence protein, can be incorporated concurrently without distorting their functions (Gegos, J. A. et al., Cell 103, 609-620 (2000); Chalfie, M. & Kain, S., Eds. (1998) GFP: Green Fluorescent Protein Properties, Applications and Protocols (Wiley-Liss, New York)).

As will be described in the following embodiment, a transgenic animal, with the gene of marker fluorescent protein incorporated, is produced. Such animal can be used to supply cells, tissues and organs that express fluorescence of the introduced gene. They can be used as valuable materials for biomedical research, such as development of transplantation therapy, investigation of cell and tissue function, and others. For example, donor cells can be identified and examined after cell or organ transplantation into recipients in medical research.

From following embodiment, which achieved production of a transgenic animal with the gene of marker fluorescent protein incorporated, it is obvious that an useful foreign gene can be also incorporated with the marker gene. If expression of the foreign gene might be effected by the gene encoding the fluorescent protein, such effect can be avoided by inserting an insulator gene, which is a boundary in a chromosome arid known to operate to blockade effect of near-existing gene.

Various kinds of mammalian transformants can be produced according to the method of this invention. The method of this invention is applicable to birds or fishes, not limited to mammals. According to this method, a foreign gene is introduced to seminiferous tubule of a male animal. Therefore, this method can be applied to any animal, so far as the animal bears seminiferous tubule or the like. The transgenic animal produced according to the method of this invention is also within the scope of this invention.

For selection of spermatocytes, spermatids or spermatozoa transfected by a foreign gene, various fluorescent proteins can be utilized as a marker. Suitable fluorescence protein may include Green Fluorescent Protein, Blue Fluorescent Protein, Yellow Fluorescent Protein, Cyan Fluorescent Protein and Red Fluorescent Protein. In addition, any marker protein, detectable in live spermatogenic cells and spermatozoa, can be also used for the same purpose.

Various foreign genes can be introduced into an animal according to the method of this invention as the intended foreign gene. Theoretically, any gene can be introduced to an animal according to the method of this invention. The breed improvement of a domestic animal can be performed by introducing a foreign gene according to the method of this invention. For example, a domestic animal bearing high quality of meat with low fat can be produced by introduction of gene encoding growth hormone. Moreover, production of a domestic animal, exhibiting tolerance to pathogenic viruses, is necessary at present. Here, character of a domestic animal can be improved to render increased tolerance to disease. Furthermore, a cow capable of secreting large amount of milk can be produced by introduction of gene involved in secretion of milk.

According to the method of this invention, an animal can be transformed to produce various useful substances. For example, a transgenic animal can be produced to obtain an useful substance or a medicine, such as insulin, growth hormone and α-antitrypsin. When some disorder occurs in said transgenic animal by increased production of physiological active substances, such disorder can be avoided by expressing the physiological active substance in mammary gland cell, so as to enable immediate secretion of said physiological active substance from the body.

At present, production of an animal expressing human tissue histocompatibility antigen gene and utilization of the animal as a donor of organ transplantation have been examined. According to the method of this invention, tissue histocompatibility antigen gene of the donor animal can be deleted or substituted by corresponding gene derived from human. It would result in production of an animal exhibiting excellent characteristic as a donor animal for organ transplantation.

### EXAMPLES

### (Preparation of plasmid DNA)

To EcoRI site of pCAGGS vector, containing cytomegalovirus enhancer, chick beta actin promoter, and rabbit beta globin polyadenylation signal, EYFP-Mito gene originated from NheI-NotI fragment of pEYFP-Mito vector (clontech) was subcloned and pCAG-EYFP-Mito expression vector was thus constructed. After transfection of Escherichia coli JM109 by the pCAG-EYFP-Mito expression vector and cultivation of the strain, the vector was prepared using QIAGEN Plasmid Mega Kit (QIAGEN). CAG-EYFP-Mito gene, utilized for introduction into mouse germ cells, the gene was isolated from pCAG-EYFP-Mito vector using restriction enzyme Sal I and Hind III.

### (DNA injection and electroporation)

Postnatal day 14 ICR strain mice was anesthetized by nembutal solution and testis was exposed under a dissecting microscope. A micro-pipette was inserted into the rete testis for injection into seminiferous tubules. Approximately 6-10 µl of the DNA/HBS (HBS: Hanks' balanced salt) solution (100-120 µg/ml) was injected into each testis.

After injection of DNA, electronic pulses were delivered with an electric pulse generator (electro square porator T820; BTX, San Diego, California). The electric pulse generator was connected with a pulse analyzer (optimizer 1500; BTX) for detection of the pulses. The direction of the pulses were also controlled to plus direction or minus direction using a resistance measure equipped with a monitor output (MBX-4, TRTECH Co., Ltd.). Testes were held between a pair of tweezers-type electrodes (diameter of 10 mm) and square electronic pulses were applied 4 times in the reverse directions, namely 4 times in the plus direction and 4 times in the minus direction, at 30-50 v/cm. Duration of each pulse was 50 msec. After electronic pulse treatment described above, muscle and skin were sutured. The mice was bred until sampling of germ cells used for further analysis or intra-cytoplasmic injection.

### (Sampling of sperm)

From male mice (ICR) transfected with CAG-EYFP-Mito DNA, testes were isolated 18-20 days after electronic pulse treatment. Seminiferous tubules, containing YFP expressing sperm, were extracted by tweezers using a microscope equipped with an excitation light source and appropriate filter sets for YFP. They were put on-slides glass, then YFP expressing sperm were examined under excitation of 488 nm using a fluorescence microscope. Seminiferous tubules containing YFP expressing sperms were placed in cooled Dulbecco's PBS supplemented with 5.6 mM glucose, 5.4 mM sodium lactate and 1% PVP (polyvinylpyrrolidone: molecular weight 360,000, Wako Pure Chemical Industries, Ltd.). Samples were cut into small pieces with scissors and dispersed in 0.5 mg/ml trypsin in PBS. Sperm and other spermatogenic cells were dispersed into the medium by gentle pipetting. The cell suspension was washed twice with Hepes-CZB (CZB: see, J. Reprod. Fertil. 86, 679, 1989).

### (Preparation of oocyte)

Ovulation of B6D2F1 female mice (age: 7-10 weeks) was induced by administration of serotropin (Teikoku Hormone Mfg. Co., Ltd.) and gonadtropin (Teikoku Hormone Mfg. Co., Ltd.). Oocytes were extracted from oviduct 16-18 hours after injection of gonadtropin. The oocytes were isolated by treating with 0.1 % hyaluronidase (440 U/mg: Sigma Chemicals) dissolved in Hepes-CZB under existence of 5% carbon dioxide for 2 minutes at 37°C.

### (Injection of a spermatozoon to an oocyte)

The isolated spermatozoa were transferred to a microdrop (2 µl) of 12 % PVP in Hepes-buffered CZB solution. Expression of YFP was detected under a fluorescence microscope (BP 470-490 and BA515) (LEICA MI APO Leica) equipped with a standard filter unit. YFP-positive spermatozoa and YFP-negative spermatozoa were separated. A YFP-positive spermatozoon was isolated by aspirating into an injection pipette attached to a piezo electric pipette-driving unit. After the sperm tail was separated from the head by applying once or several times of piezo pulses to the neck region of the sperm, the sperm head was injected into an oocyte. All injection operations were performed in Hepes-CZB within 1 hour at room temperature to avoid dehydration of sperm. Sperm-injected oocytes were incubated in CZB at 37.5 °C with 5 % carbon dioxide for 4-6 hours and then the oocytes were subjected for examination. Oocytes having two pronuclei and a secondary polar body were considered to be fertilized normally.

### (Embryo transfer)

In the fertilized ovum, a secondary polar body and two pronuclei were observed after 4-6 hours of incubation. ICR female mice and male sterile mice were mated to prepare pseudo-pregnant condition and produced zygotes were transferred to oviduct of the female mice after 18 hours. Approximately 10-15 embryos were transferred to each oviduct. A female recipient mouse was dissected at the 12.5th day of pregnancy and YEP expression of fetuses was examined using a fluorescence microscope under bright field or under excitation light. Other female recipients were subjected to Caesarean section at the 19.5-20.5th day of pregnancy. The living fetuses were brought up by a foster mother.

### (Histochemical analysis)

Testes and embryos were fixed in 2.5 % formaldehyde at 4 °C for 2-4 hours. Using a microscope equipped with an excitation light source for YFP and appropriate filter, YFP-positive seminiferous tubules were taken out with tweezers. They were put on slides glass. Also, YFP-positive germ cells were examined by a confocal laser microscope. Examination was performed under excitation wavelength of 488 nm with band pass filter of 510 to 525 nm.

### (Southern blotting analysis)

DNA of fetuses (the 12.5th day of fertilization), developed from YFP-positive sperm, were analyzed by Southern blotting. Genomic DNA was digested by restriction enzyme Apa I, and applied to electrophoresis using 1.0 % agarose gel, then transferred to blotting membrane filter (Hybond-N+ nylon membrane filter). Subsequently it was hybridized at 65 °C for 18 hours, using EYFP gene labeled with ³²P-dCTP, as a probe (740 bp, BamH I/Not I fragment). Washing of the filter was performed in the following order and analyzed by BAS2000 image analyzer. That is, (1) 2 x SSC / 0.1 % SDS (1 x SSC = 0.15M NaCl, 0.015 M sodium citrate, pH 7.0, SDS: sodium dodecyl sulfate), room temperature; (2) 1 x SSC / 0.1 % SDS, 65°C; (3) 0.5 x SSC / 0.1 % SDS, 65 °C; (4) 0.1 x SSC / 0.1 % SDS, 6°C.

### (Confirmation of gene introduction)

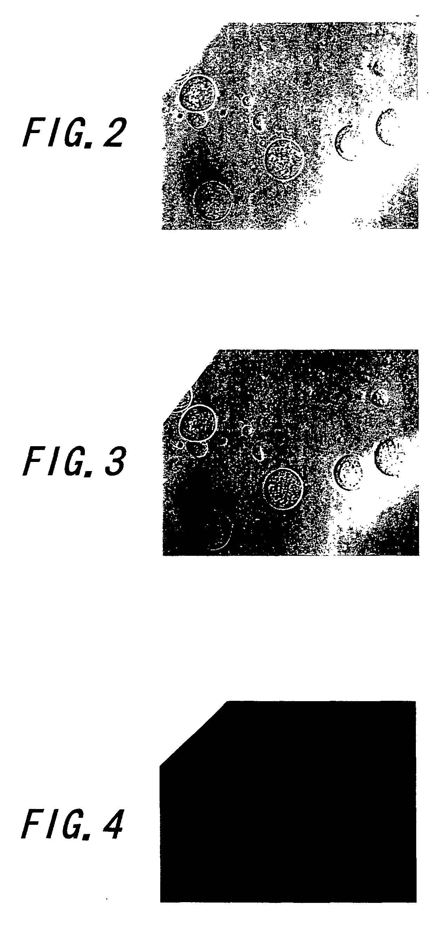

Conditions for gene introduction were optimized by altering various parameters for electroporation, such as DNA concentration, voltage, time, and number of pulses. Introduction of GFP/YFP gene was confirmed using fluorescence as a marker. Cells were separated from testis 15-18 days after introduction of GFP/YFP gene and existence of fluorescent spermatocytes and spermatids was investigated. The results are shown in Figs. 2-7.

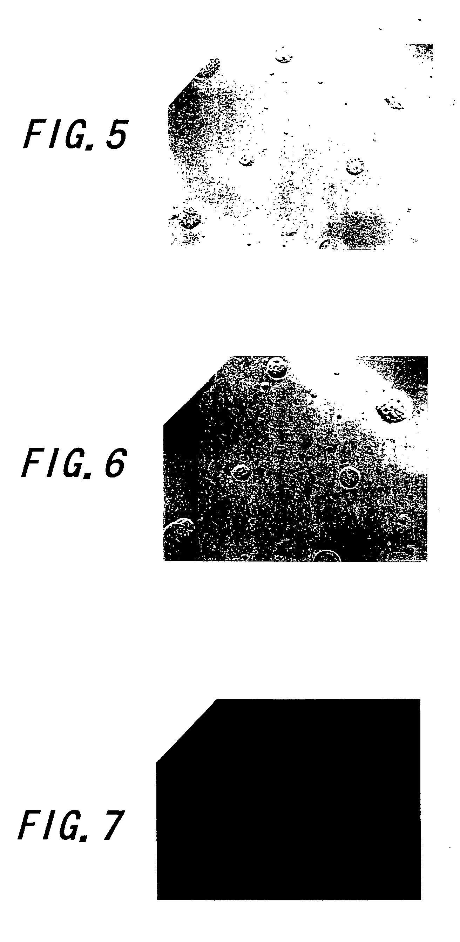

Figs. 2-4 and Figs. 5-7 show the results of spermatocytes and spermatids, respectively. Figs. 2 and 5, Figs. 3 and 6, and Figs. 4 and 7 show the bright field image, the fluorescence image and the overlapping image of bright field and fluorescence, respectively. From result of Figs. 6 and 7, existence of cells, transfected by GFP gene and differentiated to spermatid, was confirmed 15-18 days after gene introduction.

### (Expression of GFP/YFP gene in testis)

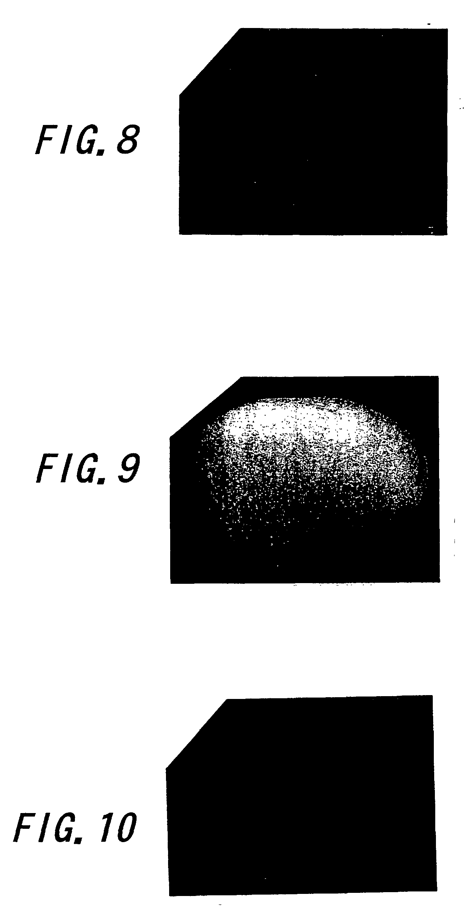

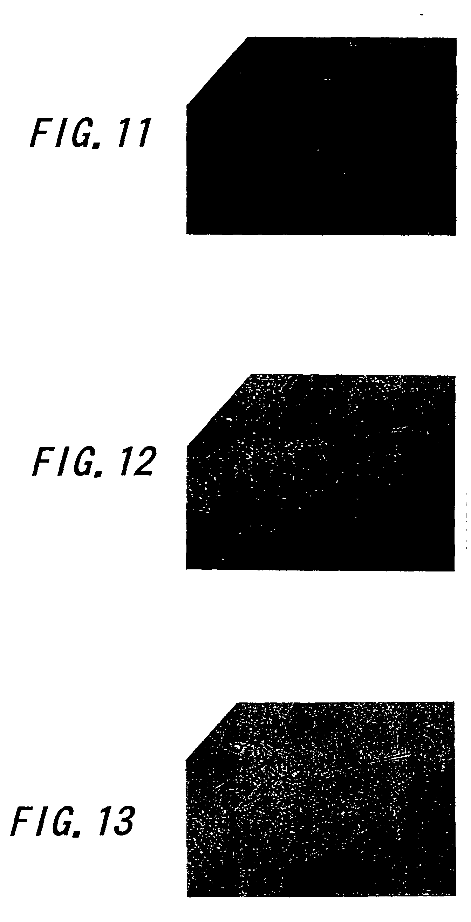

Expression of GFP/YFP gene in testis and seminiferous tubule was examined. Figs. 8 and 9 are photographs of testis 2 days after gene introduction. Figs. 8 and 9 show fluorescence image and bright field image, respectively. Figs. 10 and 11 are fluorescence images of seminiferous tubule. Figs. 10 and 11 show seminiferou tubules 2 days and 18-20 days after gene introduction, respectively. Figs. 12 and 13 show spermatozoa isolated from seminiferous tubule.

In this invention, transfection of germ cells existing in testis was performed as early as possible, so that the gene can be introduced into immature spermatogenic cells at early stages of development. Since opening of central cavity in seminiferous tubule occurs at the age of 13 days or 14 days, gene can be injected into seminiferous tubules after this stage. The histochemical examination showed that there were only spermatogonia and primary spermatocytes in testis of 13-14 days old mice.

Widespread expression of GFP/YFP fluorescence was detected in germ cells and Sertoli cells (Fig. 8). Although the number of germ cells expressing fluorescence did not alter for 7 days after electroporation but the number decreased drastically during the following week. It was shown that Sertoli cells maintained strong fluorescence, existing scattered along the seminiferous tubule as single cells. In contrast, the remaining fluorescent germ cells were present mostly as clusters of 20 to 50 cells. As the result of analysis on dissociated seminiferous tubule, fluorescence was detected in primary spermatocytes, spermatids and spermatozoa (Fig. 10 and Fig. 11). They existed as groups of several clusters and consisted of small number of spermatocytes, spermatids and spermatozoa.

In testis examined 18-20 days after gene introduction, existence of cluster of fluorescent spermatozoa was observed in seminiferous tubule (Fig. 12). The occurrence of such cluster of sperm was comparatively rare. It suggested either that gene introduction was comparatively rare or GFP/YFP might be toxic. Actually, germ cells, bearing some abnormality and fluorescence and degenerating, were observed in testis 14 days after gene introduction using GFP. Such toxicity of GFP was also reported on cultured cells. In this invention, in the purpose to reduce such toxicity, YFP linked to mitochondrial localization signal was used. As the result, spermatozoa expressing fluorescence were obtained only when such YFP was used.

### (Development of oocyte fertilized by intra-cytoplasmic sperm injection)

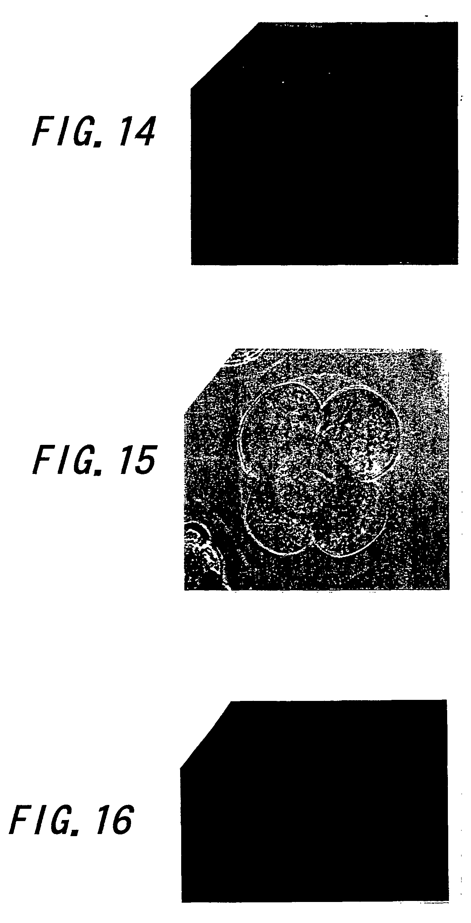

Fragment of seminiferous tubule containing fluorescent sperm was dissected and they were allowed to dissociate with trypsin solution.
The fluorescent spermatozoa were picked up and they were injected into oocytes. The result of development of such produced zygotes according to this method was summarized in table 1. The numbers described in table 1 indicate the numbers of fertilized oocytes, early embryos, fetuses and pups, respectively. At first, 3 fertilized oocytes were cultivated for 48-72 hours to produce 8-cell stage embryos. Figs. 14 and 15 show fluorescence image and bright field image, respectively. All 8-cell stage embryos mentioned above exhibited expression of fluorescent protein, as shown in Fig. 14 (Table 1, D of Example 1).

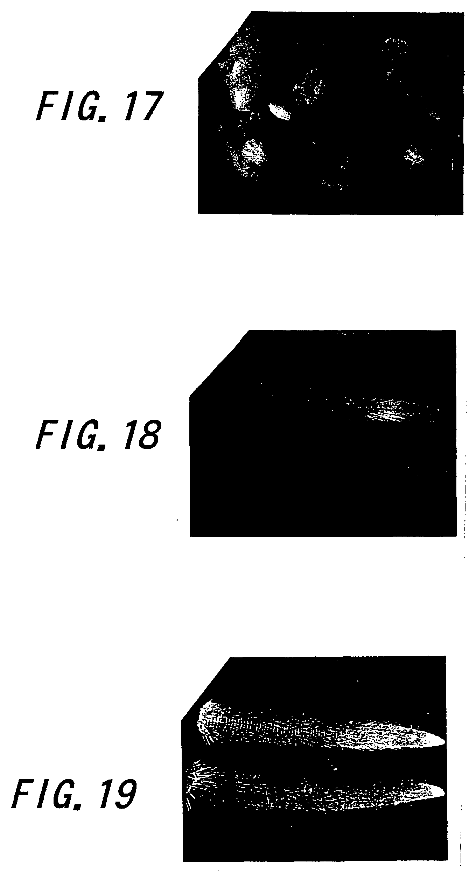

Next, 11 fertilized oocytes (5 oocytes were transferred to one foster mother mouse and 6 oocytes were transferred to another foster mother mouse) were transferred to oviducts of the two foster mother mice (Table 1, Example 2). As the result, pregnancy of the two foster mother mice was confirmed and transferred embryos developed to fetuses. Three fetuses grown in the foster mother mouse with 5 oocytes transferred were examined at the 12.5th day. Figs. 16 and 17 show fluorescence image and bright field image of these fetuses, respectively. Consequently, overall expression of YEP was observed in the body of 2 fetuses among 3 fetuses, as shown in Fig. 16 (Table 1, E of Example 2).

Furthermore, 2 alive pups (F0 generation) were obtained from another foster mother mouse with 6 oocytes transferred and the two pups expressed fluorescence of YEP (Table 1, F of Example 2). Fluorescence image examined using tails of obtained individuals is shown in Fig. 18 and bright field image of that is also shown in Fig. 19, respectively. As shown in Fig. 18, fluorescence was observed in overall tails. In Example 3, 6 fertilized oocytes were transferred to another foster mother. As the result, all of 3 pups born expressed fluorescence.

Furthermore, transmission of the transgene, from the transgenic F0 mice into the F1 generation, was confirmed. The F1 generation born from male and female transgenic F0 mice was obtained and investigation was performed. The ratio of fluorescent to non- fluorescent F1 pups was approximately 1:1 (Table 1, G of Example 2 and Example 3). This result indicates that transgene introduced into a transgenic animal produced by the method of this invention is inherited to the offspring. Moreover, there was no apparent indication of toxicity or disadvantage of the transgenic offspring compared to non-transgenic individuals.

**Table 1**

| | Example 1 | Example 2 | | Example 3 |
|---|---|---|---|---|
| A. Sperm-injected oocytes | 3 | 18 | | 11 |
| B. Oocytes with sperm pronuclei | 3 | 11 | | 6 |
| C. One-cell stage embryos transferred to foster mother | - | 5 | 6 | 6 |
| D. Fluorescent early embryos / total embryos | 3/3 | - | - | - |
| E. Fluorescent fetuses / implanted fetuses | - | 2/3 | - | - |
| F. Fluorescent pups (F0) / pups born | - | - | 2/2 | 3/3 |
| G. Fluorescent pups (F1) / pups born by mating of F0 animals | - | - | 5/8 4/7 | 7/12 3/7 |

### (Confirmation of integration of transgene into chromosome)

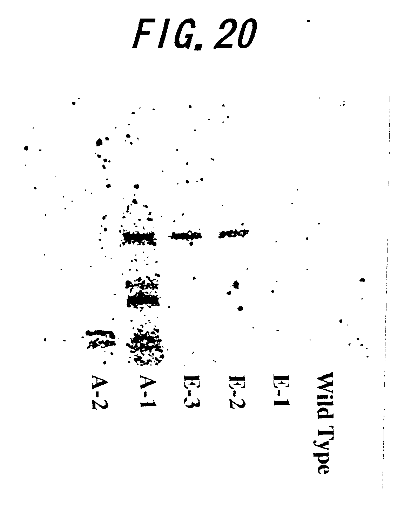

Southern blotting was performed using DNA samples derived from fetuses and pups produced by intra-cytoplasmic sperm injection. In Fig. 20, lanes E-1 to E-3 indicate the result of analysis performed on samples of fetuses. E-1 is the result of fetus developed from non-fluorescent sperm and E-2 and E-3 are those developed from fluorescent sperm. In E-2 and E-3, a band corresponding to YFP gene was recognized in the middle region of the blotting figure. Therefore, integration of the transgene was confirmed.

In Fig. 20, lanes of A-1 and A-2 show results analyzed on samples of born pups. Both of A-1 and A-2 are results analyzed on pups born from fluorescent sperm. A band corresponding to YFP gene was recognized in the upper region of the blotting figure, also indicating integration of the transgene. YFP gene was detected as single or multiple copies. From the result described above, it was indicated that the introduced gene was integrated into chromosomes of fetuses.

This invention provides a novel method for production of a transgenic animal. The method comprises steps of introducing a foreign gene into testis of an animal, selection of spermatocytes, spermatids and spermatozoa with the foreign gene introduced using marker gene as an indicator, followed by fertilization with an unfertilized oocyte or ovum.
Since this method enables selection of spermatocytes, spermatids and spermatozoa transfected by the intended gene, a transgenic animal can be produced efficiently at low cost.

## Claims

1. A method for production of a transgenic animal with a gene encoding a fluorescent protein introduced, the method comprising the steps of:
preparing a plasmid into which said gene encoding a fluorescent protein is incorporated in the plasmid ;
introducing said plasmid into germ cells existing in a testis of an animal by electroporation method or lipofection method to prepare transfected spermatocytes, spermatids or spermatozoa in which said gene encoding a fluorescent protein is introduced;
separating said transfected spermatocytes, spermatids or spermatozoa from non-transfected spermatocytes, spermatids or spermatozoa using fluorescence of said fluorescent protein as a marker ;
fertilizing an unfertilized oocyte or ovum with said transfected spermatocyte, spermatid or spermatozoon to prepare a fertilized zygote ; and
obtaining a transgenic animal individual from said fertilized zygote.

2. A method for production of a transgenic animal with an intended foreign gene introduced, the method comprising the steps of :
preparing a plasmid into which said intended foreign gene and a gene encoding a fluorescent protein are incorporated in the plasmid ;
introducing said plasmid into germ cells existing in a testis of an animal by electroporation method or lipofection method to prepare transfected spermatocytes, spermatids or spermatozoa in which said intended foreign gene and said gene encoding a fluorescent protein are introduced;
separating said transfected spermatocytes, spermatids or spermatozoa from non-transfected spermatocytes, spermatids or spermatozoa using fluorescence of said fluorescent protein as a marker;
fertilizing an unfertilized oocyte or ovum with said transfected spermatocyte, spermatid or spermatozoon to prepare a fertilized zygote; and
obtaining a transgenic animal individual from said fertilized zygote.

3. The method according to claim 1 or claim 2, wherein said animal is selected from the group consisting of mammals, birds and fishes.

4. The method according to claim 1 or claim 2, wherein said fluorescent protein is selected from a group consisting of Green Fluorescent Protein, Blue Fluorescent Protein, Yellow Fluorescent Protein, Cyan Fluorescent Protein and Red Fluorescent Protein.

5. The method according to claim 1 or claim 2, wherein said fluorescent protein is linked to mitochondrial localization signal peptide to reduce toxicity of said fluorescent protein.

6. The method according to claim 2, wherein said intended foreign gene is transmitted to offspring of said transgenic animal individual.

7. A transgenic animal produced by method according to claim 1 or claim 2.
